(19) Europäisches Patentamt
European Patent Office
Office européen des brevets

(11) **EP 4 502 137 A1**

(12) **EUROPEAN PATENT APPLICATION**
published in accordance with Art. 153(4) EPC

(43) Date of publication:
**05.02.2025 Bulletin 2025/06**

(21) Application number: 23780872.0

(22) Date of filing: **30.03.2023**

(51) International Patent Classification (IPC):
*C12N 1/16* (2006.01)    *C12Q 1/06* (2006.01)
*G01N 33/02* (2006.01)    *C12M 1/34* (2006.01)

(52) Cooperative Patent Classification (CPC):
**C12Q 1/04; C12M 1/34; C12M 41/36; C12Q 1/06;**
**G01N 33/02;** C12N 1/16

(86) International application number:
**PCT/JP2023/013171**

(87) International publication number:
**WO 2023/190864 (05.10.2023 Gazette 2023/40)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **31.03.2022 JP 2022059578**

(71) Applicant: **Nippon Shinyaku Co., Ltd.**
**Kyoto-shi**
**Kyoto 601-8550 (JP)**

(72) Inventors:
• **TSUDA Kentaro**
  **Kyoto-shi, Kyoto 601-8550 (JP)**
• **TAMIYA Hisato**
  **Kyoto-shi, Kyoto 601-8550 (JP)**

(74) Representative: **J A Kemp LLP**
**80 Turnmill Street**
**London EC1M 5QU (GB)**

(54) **METHOD FOR ESTIMATING MICROBIAL GROWTH RATE IN FOOD, METHOD FOR EVALUATING SHELF LIFE OF FOOD, AND SYSTEM FOR ESTIMATING MICROBIAL GROWTH RATE IN FOOD**

(57)    For providing a method for estimating a microbial growth rate in food that is capable of rapidly and nondestructively evaluating shelf life of food (e.g., best-by date) and the like while reducing cost and labor associated with the evaluation, the present disclosure includes: a step of placing a hydrophilic membrane filter on a surface of a food sample, and inoculating microorganisms on the hydrophilic membrane filter; a step of imaging the microorganisms growing on the hydrophilic membrane filter over time using a microscope; a step of digitizing area of the microorganisms from the image taken over time; and a step of estimating a growth rate of the microorganisms based on the digitized data.

FIG.1

**Description**

TECHNICAL FIELD

The present disclosure relates to a method for estimating a microbial growth rate in food, a method for evaluating shelf life of food, and a system for estimating the microbial growth rate in food that are capable of rapidly evaluating shelf life of food (e.g., best-by date) and the like.

BACKGROUND ART

For example, shelf life (e.g., best-by date) of solid food such as ham is typically evaluated by a method (colony counting method) including using the food as a sample, inoculating microorganisms to the sample, storing the sample for a predetermined time, grinding and diluting the sample, placing the sample in a Petri dish, pouring an agar medium to the Petri dish for plate culture for approximately 2 days, counting colonies formed by the microorganisms to measure a growth rate of the microorganisms, and evaluating the shelf life.

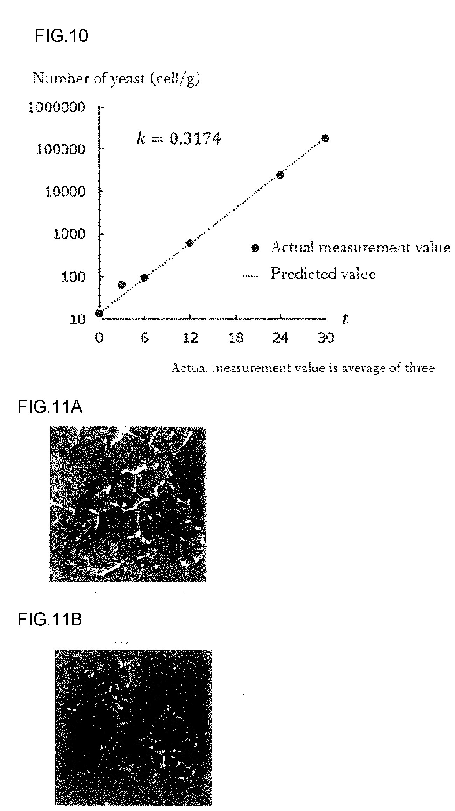

FIGs. 11A and 11B are photomicrographs taken when microorganisms (yeast) are inoculated on a surface of ham. FIG. 11A shows the surface of ham at 0 hours from the inoculation, and FIG. 11B shows the surface of ham at 18 hours from the inoculation. As shown in the figures, it is difficult to discriminate which part is the microorganisms (yeast) even when the surface of ham is directly observed with a microscope. Thus, the method as described above has been generally employed.

However, the method described above requires culture of a sample prepared by grinding food and diluting the resultant. Thus, there are the following problems, for example: (1) labor is required for grinding food, and a new solid sample is required for each measurement point for grinding of the food; (2) because the dilution should be conducted such that the number of colonies is suitable for counting (such that the number of cells is approximately 30 to 300 per 1 g of diluent), it is difficult to control dilution magnification for achieving such condition; and (3) time is required for culturing the sample until suitable colony formation and colony numbers for counting are achieved, and also time for counting is required. In particular, the problem (2) leads to increase of cost and labor because there is no clue of how many number of cells exist, and thus it becomes necessary to prepare and evaluate many stepwisely-diluted suspensions, and the problem (3) exerts a huge influence on time, labor, and the like. Thus, an alternative method has been desired for a long time.

Under such circumstances, there have been various proposals as a method for rapidly counting microorganisms in food, including inoculating microorganisms to a sample, storing and then diluting the sample as described above, filtering the sample with a membrane filter to capture and fix the microorganisms, coloring the microorganisms with a fluorescent dye or the like, and counting the number of microorganisms by an image analyzer, a laser scanning device, or the like (see PTLs 1 and 2, and the like).

RELATED ART DOCUMENT

PATENT DOCUMENT

PTL 1: JP-A-2000-210099
PTL 2: JP-A-2007-020528

SUMMARY

PROBLEMS TO BE SOLVED BY THE DISCLOSURE

However, the methods disclosed in PTLs 1 and 2 require staining of microorganisms with a fluorescent dye or the like. Thus, cost is required for the agent.

Further, even when the methods disclosed in PTLs 1 and 2 are applied to evaluate the shelf life of food, processes of grinding and diluting the food are required, and thus there is room for examination on this point. In other words, conventionally, a method for non-destructively and rapidly evaluating the shelf life of solid food has not been sufficiently established, and thus there is room for studying such a method.

The present disclosure was made in view of such circumstances, for providing a method for estimating a microbial growth rate in food, a method for evaluating shelf life of food, and a system for estimating the microbial growth rate in food that are capable of non-destructively and rapidly evaluating shelf life of food (e.g., best-by date) and the like, while reducing cost and labor associated with the evaluation.

MEANS FOR SOLVING THE PROBLEMS

The present inventors have made intensive study for solving the above problems. During the study, the present inventors considered that if microorganisms present on a surface of food can be directly observed without performing treatment such as fixation and staining after filtration as described above, a growth rate of the microorganisms growing on the surface of the food can be estimated, and thus shelf life of food and the like can be evaluated non-destructively.

However, it is difficult to directly observe microorganisms present on the surface of food without performing fixation, staining, or the like, due to problems such as the light transmissivity and surface structure of the food.

The present inventors have further studied, and as a result, they found that when a hydrophilic membrane filter is placed on the surface of food and microorganisms are inoculated on the hydrophilic membrane filter, the hydrophilic membrane filter serves as a carrier (bed), and thus a degree of growth of the microorganisms can be easily observed. Also, they found that the growth rate of the microorganisms inoculated on the hydrophilic membrane filter placed on the surface of the food is not different from the growth rate of microorganisms directly inoculated on the surface of the food.

They further found that the growth rate of microorganisms growing on the surface of food can be estimated by taking a photomicrograph of the microorganisms inoculated on the hydrophilic membrane filter over time, digitizing area occupied by the microorganisms for each photomicrograph, calculating a degree of change in the numerical values (coefficient) over time, and substituting the obtained value into a predetermined prediction formula.

By the above finding, it becomes possible to rapidly predict time required for the number of microorganisms to reach a predetermined number that is not acceptable in the food without requiring a step of grinding food, and the like, and also without performing culture until suitable colony formation and colony numbers for counting are achieved, as in the conventional colony counting method. That is, it becomes possible to evaluate the shelf life of the food (e.g., best-by date) and the like, non-destructively and rapidly.

That is, the present disclosure includes the following aspects [I] to [IX].

[I] A method for estimating a microbial growth rate in food including: a step of placing a hydrophilic membrane filter on a surface of a food sample, and inoculating a microorganism on the hydrophilic membrane filter; a step of imaging the microorganism growing on the hydrophilic membrane filter over time using a microscope; a step of digitizing area of the microorganism from the image taken over time; and a step of estimating a growth rate of the microorganism based on the digitized data.

[II] The method for estimating a microbial growth rate in food as recited in [I], wherein the step of digitizing the area of the microorganism is performed by image analysis using a computer.

[III] The method for estimating a microbial growth rate in food as recited in [I] or [II], wherein the step of estimating the growth rate of the microorganism is performed by calculation using a computer.

[IV] A method for evaluating shelf life of food including: a step of placing a hydrophilic membrane filter on a surface of a food sample, and inoculating a microorganism on the hydrophilic membrane filter; a step of imaging the microorganism growing on the hydrophilic membrane filter over time using a microscope; a step of digitizing area of the microorganism from the image taken over time; a step of estimating a growth rate of the microorganism based on the digitized data; and a step of predicting time required for a number of microorganism to reach a predetermined number that is not acceptable in the food using the estimated growth rate.

[V] The method for evaluating shelf life of food as recited in [IV], wherein the step of digitizing the area of the microorganism is performed by image analysis using a computer.

[VI] The method for evaluating shelf life of food as recited in [IV] or [V], wherein the step of estimating the growth rate of the microorganism is performed by calculation using a computer.

[VII] A system for estimating a microbial growth rate in food, the system including: an imaging device including a microscope; a digitization device capable of digitizing area of a microorganism on a subject from an image of the subject taken by the imaging device over time; and an estimation device capable of estimating a growth rate of the microorganism from an amount of change in the area over time, wherein the subject is prepared by placing a hydrophilic membrane filter on a surface of a food sample, and inoculating a microorganism on the hydrophilic membrane filter.

[VIII] The system for estimating a microbial growth rate in food as recited in [VII], wherein the digitization device is an image analyzer using a computer.

[IX] The system for estimating a microbial growth rate in food as recited in [VII] or [VIII], wherein the estimation device is a calculation device using a computer.

EFFECTS OF THE DISCLOSURE

The method for estimating a microbial growth rate according to the present disclosure can evaluate shelf life of food (e.g., best-by date) and the like non-destructively and rapidly, while reducing cost and labor. Further, the system for estimating a microbial growth rate according to the present disclosure can estimate a growth rate of microorganisms in food non-destructively and rapidly, while reducing cost and labor.

BRIEF DESCRIPTION OF DRAWINGS

FIG. 1 is an explanatory view showing an exemplary system for measuring and evaluating kinetics of microorganisms inoculated on a food sample.

FIG. 2 is an explanatory view showing the state where microorganisms are inoculated to the food sample.

FIG. 3A is a photomicrograph showing kinetics observed when yeast cells are inoculated on a hydrophilic membrane filter placed on an agar medium, at 0 hours from the inoculation.

FIG. 3B is a photomicrograph showing kinetics observed when yeast cells are inoculated on a hydrophilic membrane filter placed on an agar medium, at 9 hours from the inoculation.

FIG. 4A is an image-processed photomicrograph showing kinetics observed when yeast cells are inoculated on the hydrophilic membrane filter placed on the agar medium, at 0 hours from the inoculation.

FIG. 4B is an image-processed photomicrograph showing kinetics observed when yeast cells are inoculated on the hydrophilic membrane filter placed on the agar medium, at 9 hours from the inoculation.

FIG. 5 is a graph showing a change rate in area of yeast cells at each hour in Example 1.

FIG. 6 is a graph showing a prediction formula indicating a change rate in the number of yeast at each hour in Example 1, and plots of actually measured number of yeast in the conventional method.

FIG. 7A is a photomicrograph of Example 2, at 0 hours from the inoculation.

FIG. 7B is a photomicrograph of Example 2, at 12 hours from the inoculation.

FIG. 8A is an image-processed photomicrograph of Example 2, at 0 hours from the inoculation.

FIG. 8B is an image-processed photomicrograph of Example 2, at 12 hours from the inoculation.

FIG. 9 is a graph showing a change rate in area of yeast cells at each hour in Example 2.

FIG. 10 is a graph showing a prediction formula indicating a change rate in the number of yeast at each hour in Example 2, and plots of actually measured number of yeast in the conventional method.

FIG. 11A is a photomicrograph of the surface of food (ham) to which yeast cells are inoculated, at 0 hours from the inoculation.

FIG. 11B is a photomicrograph of the surface of food (ham) to which yeast cells are inoculated, at 18 hours from the inoculation.

EMBODIMENTS OF THE DISCLOSURE

Hereinafter, the embodiments of the present disclosure will be described in detail. However, the present disclosure is not limited to these embodiments.

In the present disclosure, the expression "X to Y" (X and Y are given numbers), unless otherwise specified, encompasses the meanings of "not less than X and not greater than Y," and "preferably greater than X" or "preferably less than Y."

The expression "not less than X" (X is a given number) or "not greater than Y" (Y is a given number) encompasses the meaning of "preferably greater than X" or "preferably less than Y."

The method for estimating a microbial growth rate in food according to one embodiment of the present disclosure (hereinafter, referred to as "the present estimation method") includes a step of placing a hydrophilic membrane filter on a surface of a food sample, and inoculating microorganisms on the hydrophilic membrane filter (step A); a step of imaging the microorganisms growing on the hydrophilic membrane filter over time using a microscope (step B); a step of digitizing area of the microorganisms from the image taken over time (step C); and a step of estimating a growth rate of the microorganisms based on the digitized data (step D).

The method for evaluating shelf life of food according to one embodiment of the present disclosure (hereinafter, referred to as "the present evaluation method") includes: a step of placing a hydrophilic membrane filter on a surface of a food sample, and inoculating microorganisms on the hydrophilic membrane filter (step A); a step of imaging the microorganisms growing on the hydrophilic membrane filter over time using a microscope (step B); a step of digitizing area of the microorganisms from the image taken over time (step C); a step of estimating a growth rate of the microorganisms based on the digitized data (step D); and a step of predicting time required for the number of the microorganisms to reach a predetermined number that is not acceptable in the food using the estimated growth rate (step E).

The "food sample" used herein is not particularly limited as long as a hydrophilic membrane filter can be placed thereon (a sample that does not exhibit a liquid state may be used). Examples thereof include a sample that is collected from food itself and exhibits a state of solid, gel, or cream; a solid medium (sample) such as an agar medium in which food components are reflected; and a solid medium (sample) prepared by solidifying liquid food (beverages or the like) with agar and the like.

The "imaging over time" means that imaging is performed at every elapse of a predetermined time, and the predetermined time does not need to be constant. Further, the "imaging over time" includes not only intermittent imaging but also continuous imaging.

Hereinbelow, each of the step (steps A to E) will be described in detail.

Method for Estimating Microbial Growth Rate

Step A

In the step A, a hydrophilic membrane filter is placed on the surface of a food sample, and microorganisms are inoculated on the hydrophilic membrane filter.

The food sample is, for example, a sample directly collected from food, and thus there is no need to grind and dilute food, or the like. This is advantageous in reducing cost and labor.

The size of the food sample is not particularly limited as long as there is no hindrance in microscopic observation.

Water activity (Aw) of the food sample is typically 0.60 to 1.00, preferably 0.80 to 1.00, and more preferably 0.90 to 1.00 from the viewpoint that nutrients or the like (growth source of microorganisms) contained in the food sample can be sufficiently transferred onto the hydrophilic membrane filter with water.

Examples of the hydrophilic membrane filter that may be used in the step A include resin membrane filters such as polytetrafluoroethylene, polyvinylidene fluoride, and polyether sulfone that are subjected to hydrophilization treatment as necessary. A commercially available hydrophilic membrane filter may also be used, and preferred examples thereof include Omnipore Membrane Filter JHWP01300, manufactured by Merck KGaA.

Pore size of the hydrophilic membrane filter is preferably such that microorganisms to be inoculated are not passed through, but nutrients or the like (growth source of microorganisms) contained in the food sample are passed. The pore size depends on the species of target microorganisms, and is preferably 0.1 to 10.0 $\mu$m, and more preferably 0.2 to 0.7 $\mu$m.

The size of the hydrophilic membrane filter is not particularly limited as long as there is no hindrance in the microscopic observation.

On the other hand, the thickness is typically 10 to 150 $\mu$m, and preferably 50 to 100 $\mu$m. When the thickness of the hydrophilic membrane filter is within the above range, nutrients or the like (growth source of microorganisms) contained in the food sample can be efficiently transferred onto the hydrophilic membrane filter through moisture.

In the step A, examples of the microorganisms to be inoculated onto the hydrophilic membrane filter include microorganisms influencing quality deterioration of target food, and microorganisms typically used in food produced by using microorganisms such as fermented food. Examples of such microorganisms include food putrefactive bacteria such as yeast, lactic acid bacteria, spore bacteria, and Escherichia coli, yeast used in bread fermentation, lactic acid bacteria used as a starter (inoculum) of yoghurt, and effective microorganisms such as Bacillus subtilis var natto. These may be used alone or in a combination.

By the present estimation method, the shelf life of target food can be evaluated by predicting a growth rate of food putrefactive bacteria, or conditions or the like for suppressing growth of the bacteria can be determined. Additionally, by the present estimation method, a growth rate of useful microorganisms can be predicted to determine conditions or the like by which the microorganisms grow properly. For example, when the useful microorganisms are yeast, a fermentation rate of the target food can be calculated.

The inoculation of the microorganisms can be performed by, for example, dropwise addition of 0.25 to 8 $\mu$L (preferably 0.5 to 4 $\mu$L, more preferably 1 to 2 $\mu$L) of the microorganisms that are diluted with water or an aqueous solution to a cell count of 50 to 2000 cells/$\mu$L (preferably 100 to 1000 cells/pL, more preferably 200 to 500 cells/pL) on the hydrophilic membrane filter.

Examples of the aqueous solution include saline, phosphate buffered saline, and peptone-added saline.

Step B

In the step B, microorganisms growing on the hydrophilic membrane filter are imaged over time using a microscope.

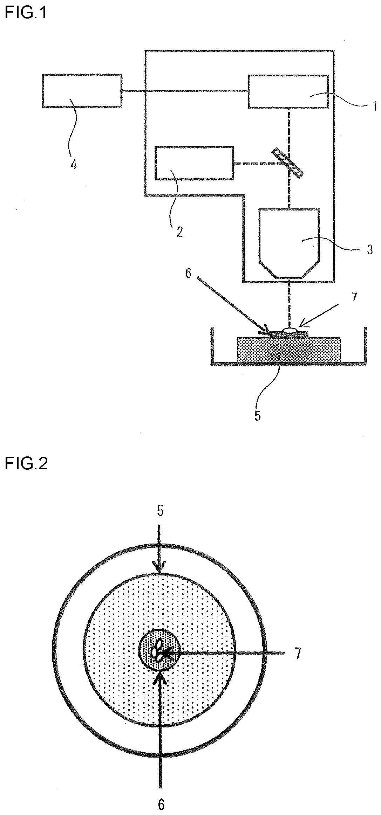

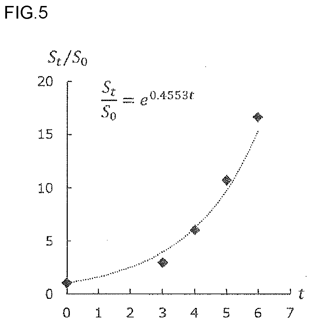

FIG. 1 shows an exemplary system for measuring and evaluating the kinetics of microorganisms inoculated on the hydrophilic membrane filter on the surface of the food sample in the present estimation method and the present evaluation method. In FIG. 1, 1 represents a camera, 2 represents a light source, 3 represents objective lens, 4 represents a personal computer, 5 represents a food sample, 6 represents a hydrophilic membrane filter, and 7 represents microorganisms. FIG. 2 schematically shows the state of microorganisms that are inoculated on the hydrophilic membrane filter on the surface of the food sample. In FIG. 2, 5 represents a food sample, 6 represents a hydrophilic membrane filter, and 7 represents microorganisms.

The camera 1, the light source 2, and the objective lens 3 represent a microscope configuration for imaging a photomicrograph. The microscope configuration is not particularly limited, and the following example is included.

Microscope Configuration

Camera: GigE camera equipped with a heatsink DMK33GX249e, manufactured by The Imaging Source, LLC
Light source: LED Spot Illumination SLSI-22W, manufactured by SIGMAKOKI Co., LTD.
Objective lens: Long Working Distance Objective Lens PAL-20, manufactured by SIGMAKOKI Co., LTD.

The imaging of microorganisms is not particularly limited, and usually performed under atmosphere temperature conditions of 4 to 45°C. The conditions may be arbitrarily set according to the purpose and the species of food and microorganisms.

A method for imaging the photomicrograph is not particularly limited. Preferably, the imaging is performed as follows: the state of microorganisms immediately after the inoculation is imaged, then the sample is imaged over time, and the imaging is completed within one-half or less (preferably one-third or less) of the time required in the conventional colony counting method using the same food sample and microorganisms are used.

As described above, the present estimation method and the present evaluation method can estimate the growth rate of microorganisms by observation in a shorter time as compared with the conventional method.

The imaging over time may be either intermittent imaging or continuous imaging, as described above. When the intermittent imaging is performed, the imaging is preferably performed every three hours, and more preferably every one hour in view of balance of estimation accuracy and an operation amount.

Step C

In the step C, area of microorganisms is digitized for each of the plurality of images taken over time. Such a process is usually performed by image analysis using a computer such as a personal computer.

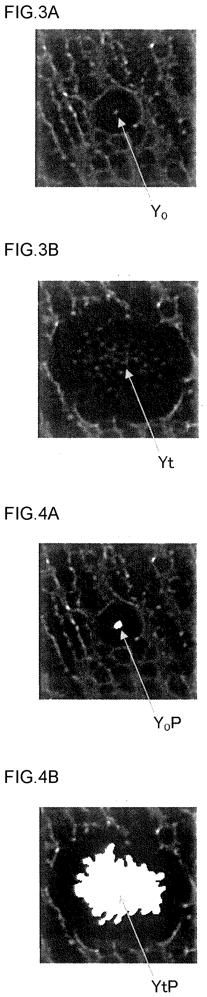

That is, as shown in FIGs. 4A and 4B, in the images taken over time, parts occupied by the microorganisms are painted using an image processing software, and area of the painted parts (white-painted parts in each figure) is calculated by the software as a pixel number (total number of pixels), thereby area of the microorganisms can be digitized from the image.

Such digitization is preferably performed by imaging the growing microorganisms in a plurality of different places on the membrane filter over time to digitize area of the microorganisms in each place, and taking the total or average of the digitized values, from the viewpoint that individual differences between microorganisms (difference in growth state of each cell) can be eliminated. The number of the plurality of places may be two, but preferably three or more, in view of obtaining more excellent accuracy.

The processing of painting the part occupied by the microorganisms on the image using the image processing software may also be performed manually by visually discriminating the cells. Alternatively, the processing may be performed automatically using a machine learning program (using support-vector machine or deep learning) in view of improving the efficiency.

Further, the steps B and C may be performed in parallel by using a microorganism detection system that is set up to perform these steps automatically.

Step D

In the step D, the growth rate of microorganisms is estimated based on the digitized data.

That is, by comparing the digitized data, change in cell image area during time "t" (time from the first imaging to the last imaging) originated from the same cell of the microorganisms is specified, and thus the plurality of data obtained over time is substituted into the following formula (1) to approximate the obtained values, thereby a coefficient k can be obtained. The formula (1) herein is based on a growth curve of microorganisms (microorganisms immediately after inoculation grow logarithmically). In the formula (1), "e" represents the base of natural logarithms (Napier's constant) (the same shall apply to the following formula (2)). The formula (1) is graphically shown in FIG. 5, in which the plurality of data is substituted into the formula (1), the obtained values are approximated, and as a result, a value of the coefficient k was determined to be 0.4553.

$$\frac{S_t}{S_0} = e^{kt} \qquad (1)$$

$S_t$: cell image area at time t
$S_0$: initial cell image area
k: coefficient
t: time

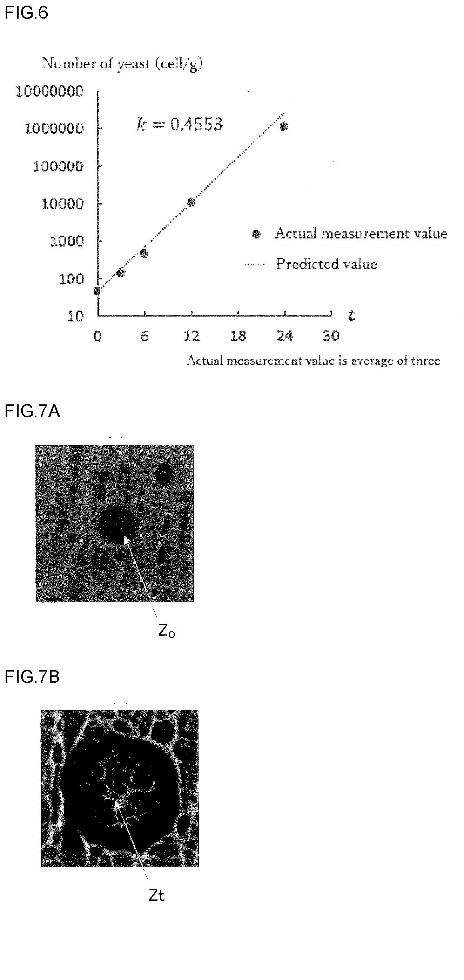

Subsequently, the value of the coefficient k calculated as above is substituted into the following formula (2), and thereby a prediction formula of the number of cells Nt at the time "t" can be obtained. FIG. 6 shows the obtained prediction formula in a semilogarithmic graph, in which actual measurement values obtained by the conventional method (colony counting method) are superimposed on the graph.

$$N_t = N_0 \, e^{kt} \qquad (2)$$

$N_t$: number of cells at time $t$
$N_0$: initial number of cells
k: coefficient
t: time

Derivation of the coefficient k and the prediction formula, or the like in the step D are preferably performed by calculation using a computer such as a personal computer in view of improving the efficiency.
The present estimation method including the above steps (steps A to D) has excellent reliability comparable to the conventional method as shown in FIG. 6, in which actual measurement values (values obtained by the conventional method) are on the prediction formula.
Further, the present estimation method can reduce observation time of the microorganisms (time from the inoculation to completion of the imaging) to one-half or less of the time required for the conventional colony counting method, as described above. The observation time can further be reduced to one-third or less by devising an atmosphere temperature or the like.

Method for Evaluating Shelf Life of Food

The present evaluation method includes the steps A to D of the present estimation method, and additionally the step E.

Step E

In the step E, time required for the number of microorganisms to reach a predetermined number that is not acceptable in food is predicted using the estimated growth rate.
Typically, the number of cells of microorganisms that is not acceptable in food (number of food putrefactive bacteria) is often set to 100000 cells/g as a reference. Thus, shelf life duration of food can be predicted by redisplaying the derived prediction formula in the linear graph of FIG. 6, in which the vertical axis represents logarithm, and calculating the time required for reaching 100000 cells/g, for example (in the graph of FIG. 6, approximately 18 hours).
Note that the number of microorganisms that is not acceptable in food may be appropriately and freely set according to the purpose.
Alternatively, the shelf life duration of food may be predicted using a computer program automatically from the derived prediction formula, rather than predicting the shelf life duration of food by redisplaying the prediction formula on another graph as described above.
The present evaluation method including the above steps (steps A to E) utilizes the present estimation method, and has excellent reliability comparable to the conventional method because actual measurement values (values measured by the conventional method) are on the prediction formula, as described above (see FIG. 6).
The present evaluation method utilizes the present estimation method, and thus has an advantage that time required for evaluating shelf life of food can be reduced to one-half or less of the time required for the conventional colony counting method, and further can be reduced to one-third or less by devising conditions and the like.
System for Estimating Microbial Growth Rate in Food
The system for estimating a microbial growth rate in food (hereinafter, may be referred to as "the present estimation system") is not particularly limited as long as the present estimation method is utilized.

From the viewpoint that the microbial growth rate in food can be efficiently estimated in a short time, the system preferably includes an imaging device including a microscope, a digitization device capable of digitizing area of microorganisms on a subject from an image of the subject taken by the imaging device over time, and an estimation device capable of estimating a growth rate of the microorganism from an amount of change in the area over time, wherein the subject is prepared by placing a hydrophilic membrane filter on a surface of a food sample and inoculating microorganisms on the hydrophilic membrane filter.

The imaging device is not particularly limited as long as it is capable of performing the step B, and examples thereof that can be used include the system described above in the present estimation method (the system including a camera, a light source, objective lens, and a personal computer).

The digitization device is not particularly limited as long as it is capable of performing the step C, and preferred is an image analyzer using a computer such as a personal computer in view of excellent versatility.

The estimation device is not particularly limited as long as it is capable of performing the step D, and preferred is a calculation device using a computer such as a personal computer in view of excellent versatility.

The present estimation system can evaluate shelf life of target food by estimating the growth rate of food putrefactive bacteria in the food, in the case where the microorganisms are food putrefactive bacteria. In addition, the estimation of the growth rate can be used for determining conditions or the like for suppressing growth of the bacteria.

On the other hand, in the case where the microorganisms are useful microorganisms, the estimation of the growth rate of the useful microorganisms in target food can be used for determining conditions or the like in which the microorganisms grow properly. For example, in the case where the useful microorganisms are yeast, a fermentation rate of target food can be estimated.

As described above, the present estimation method, the present evaluation method, and the present estimation system can evaluate shelf life of food non-destructively and rapidly, while reducing cost and labor accompanied with the evaluation and the like.

That is, the operation that takes at least several days in the conventional method can be shortened to several hours.

In addition, the present estimation method, the present evaluation method, and the present estimation system can collect data concerning shelf life of food (e.g., best-by date) and the like more rapidly and efficiently than the conventional method. Thus, it is possible to efficiently search or calculate optimum conditions (condition of adequate amount of food additives for improving shelf life or the like, species of the food additives, adjustment conditions such as acidity, salinity, and moisture content, and the like) by using a computer such as a personal computer or machine learning algorithm (e.g., neural network) based on its accumulated data.

The present estimation method, the present evaluation method, and the present estimation system can be effectively applied in business utilizing these advantages (for example, information provision of optimum conditions, or supply of materials and provision of service accompanied therewith).

EXAMPLES

Hereinafter, Examples will be described. However, the present disclosure is not limited to these Examples.

Example 1

First, an agar medium (potato dextrose agar medium, manufactured by Nissui Pharmaceutical Co., Ltd.) was prepared as a food sample, and a hydrophilic membrane filter was placed on the surface of the medium (Omnipore Membrane Filter JHWP01300, manufactured by Merck KGaA). Then, yeast (Pichia anomala) was diluted with phosphate buffered saline such that the concentration was 500 cells/2 $\mu$L, and inoculated by adding 2 $\mu$L of the diluent dropwise onto to the membrane filter (see FIG. 1).

Thereafter, the yeast growing on the membrane filter at a room temperature (25°C) was imaged over time using a microscope having the following configuration (see FIG. 1 and FIG. 2, magnification: 20 times).

Microscope Configuration

Camera: GigE camera equipped with a heatsink DMK33GX249e, manufactured by The Imaging Source, LLC
Light source: LED Spot Illumination SLSI-22W, manufactured by SIGMAKOKI Co., LTD.
Objective lens: Long Working Distance Objective Lens PAL-20, manufactured by SIGMAKOKI Co., LTD.

Examples of images taken by the imaging are shown in FIGs. 3A and 3B. FIG. 3A shows yeast $Y_0$ immediately

after the inoculation (at 0 hours), and FIG. 3B shows yeast Yt, which grew derived from the yeast $Y_0$ shown in FIG. 3A, after 9 hours from the inoculation (at 9 hours).

Then, degree of change in area occupied by yeast was calculated from the plurality of images taken over time by an image processing software, and thereby the number of cells Nt at the time "t" was predicted.

Specifically, using ImageJ (open source), the image area of yeast derived from the same cell at each hour (at 0 hours, at 3 hours, at 4 hours, at 5 hours, and at 6 hours) was painted, and the painted image area of the yeast was digitized as the number of pixels (total number of pixels). Examples of the painted images are shown in FIGs. 4A and 4B. A symbol $Y_0P$ in FIG. 4A represents a painted image area of the yeast $Y_0$ in FIG. 3A, and a symbol YtP in FIG. 4B represents painted image area of the yeast Yt in FIG. 3B.

Table 1 below shows the digitized results. Note that the yeast image area described below (cell image area) represents total of digitized area in three different places (three scenes).

[Table 1]

| Time t | 0 | 3 | 4 | 5 | 6 |
|---|---|---|---|---|---|
| Cell image area* S | 405 | 1191 | 2440 | 4344 | 6743 |

* Total value of three scenes (pixel)

Thereafter, the cell image area calculated as above was substituted into the above formula (1) to approximate the obtained values, and thereby the coefficient k was determined. As a result, the value of the coefficient k was 0.4553.

Subsequently, the value of the coefficient k was substituted into the above formula (2) to obtain a prediction formula of the number of cells Nt at the time "t."

FIG. 6 shows the prediction formula in a semilogarithmic graph, in which the number of actually measured microorganisms (number of yeast) in the conventional method (colony counting method) are superimposed. FIG. 6 shows that plots of the number of actually measured microorganisms (number of yeast) are mostly on the prediction formula in the graph. Thus, it is found that the prediction formula has the same level of high reliability as the conventional method.

As shown in FIG. 6, it is possible to estimate the time required for the number of microorganisms to reach a number that is not acceptable in food (in this case, 100000 cells/g) from the prediction formula (in this graph, approximately 18 hours).

The prediction formula can be derived in a shorter time than the conventional colony counting method as described above, and thus the evaluation of shelf life of food according to the present disclosure can be performed in a shorter time as compared with the conventional method.

Example 2

Yeast grown on a membrane filter was imaged over time in the same manner as in Example 1 except that loin ham (Morning Flesh Loin Ham, product of ITOHAM FOODS Inc.) was used as a food sample.

Examples of images taken by the imaging are shown in FIGs. 7A and 7B. FIG. 7A shows yeast $Z_0$ immediately after the inoculation (at 0 hours), and FIG. 7B shows yeast Zt, which grew derived from the yeast $Z_0$ shown in FIG. 7A, after 12 hours from the inoculation (at 12 hours).

Then, degree of change in area occupied by yeast was calculated from the plurality of images taken over time by an image processing software, and thereby the number of cells Nt at the time "t" was predicted.

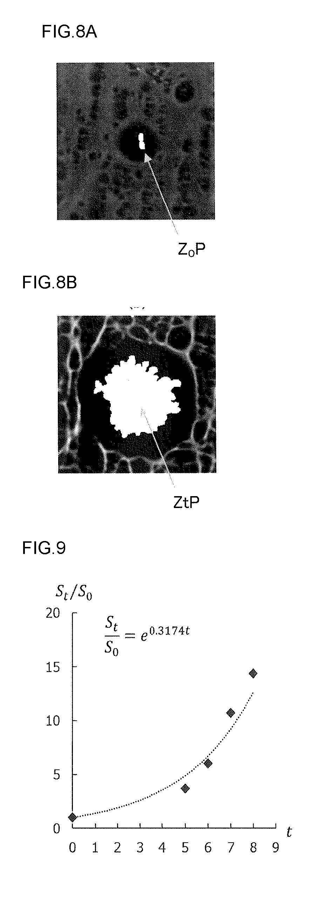

Specifically, using ImageJ (open source), the image area of yeast derived from the same cell at each hour (at 0 hours, at 5 hours, at 6 hours, at 7 hours, and at 8 hours) was painted, and the painted image area of the yeast was digitized as the number of pixels (total number of pixels). Examples of the painted images are shown in FIGs. 8A and 8B. A symbol $Z_0P$ in FIG. 8A represents a painted image area of the yeast $Z_0$ in FIG. 7A, and a symbol ZtP in FIG. 8B represents painted image area of the yeast Zt in FIG. 7B.

Table 2 below shows the digitized results. Note that the yeast image area described below (cell image area) represents total of digitized area in three different places (three scenes).

[Table 2]

| Time t | 0 | 5 | 6 | 7 | 8 |
|---|---|---|---|---|---|
| Cell image area* S | 329 | 1212 | 1987 | 3516 | 4734 |

* Total value of three scenes (pixel)

Thereafter, the cell image area calculated as above was substituted into the above formula (1) to approximate the obtained values, and thereby the coefficient k was determined. As a result, the value of the coefficient k was 0.3174.

FIG. 9 graphically shows the approximated formula (1).

Subsequently, the value of the coefficient k was substituted into the above formula (2), and thereby a prediction formula of the number of cells Nt at the time "t" was obtained.

FIG. 10 shows the prediction formula in a semilogarithmic graph, in which the number of actually measured microorganisms (number of yeast) in the conventional method (colony counting method) are superimposed. FIG. 10 shows that plots of the number of actually measured microorganisms (number of yeast) are mostly on the prediction formula in the graph. Thus, it is found that the prediction formula has the same level of high reliability as the conventional method.

As shown in FIG. 10, it is possible to estimate the time required for the number of microorganisms to reach a number that is not acceptable in food (in this case, 100000 cells/g) from the prediction formula (in this graph, approximately 28 hours).

While specific forms of the embodiments of the present disclosure have been shown in the aforementioned Examples, the Examples are merely illustrative and not limitative of the present disclosure. It is contemplated that various modifications apparent to those skilled in the art are within the scope of the present disclosure.

INDUSTRIAL APPLICABILITY

The present estimation method, the present evaluation method, and the present estimation system can evaluate shelf life of food non-destructively and rapidly, while reducing cost and labor accompanied with the evaluation and the like. That is, the operation that takes at least several days in the conventional method can be shortened to several hours. In addition, the present estimation method, the present evaluation method, and the present estimation system can collect data concerning shelf life of food (e.g., best-by date) and the like more efficiently than the conventional method. Thus, it becomes possible to efficiently search optimum conditions (for example, condition of adequate amount of food additives for improving shelf life or the like, species of the food additives, and adjustment conditions such as acidity, salinity, and moisture content) by using a computer such as a personal computer or Internet based on its accumulated big data.

Accordingly, the present estimation method, the present evaluation method, and the present estimation system can be effectively applied in business utilizing these advantages (for example, information provision of optimum conditions, or supply of materials and provision of service accompanied therewith).

REFERENCE SIGNS LIST

1 camera
2 light source
3 objective lens
4 personal computer
5 food sample
6 hydrophilic membrane filter
7 microorganism

**Claims**

1.    A method for estimating a microbial growth rate in food comprising:

placing a hydrophilic membrane filter on a surface of a sample of the food, and inoculating a microorganism on the hydrophilic membrane filter;
imaging the microorganism growing on the hydrophilic membrane filter over time using a microscope;
digitizing area of the microorganism from the image taken over time; and
estimating the microbial growth rate of the microorganism based on data from the digitized area.

2.    The method for estimating a microbial growth rate in a food according to claim 1, wherein in digitizing the area of the microorganism is performed by image analysis using a computer.

3.    The method for estimating a microbial growth rate in food according to claim 1 or 2, wherein in estimating the microbial growth rate of the microorganism is performed by calculation using a computer.

4. A method for evaluating shelf life of a food comprising:

placing a hydrophilic membrane filter on a surface of a sample of the food, and inoculating a microorganism on the hydrophilic membrane filter;
imaging the microorganism growing on the hydrophilic membrane filter over time using a microscope;
digitizing an area of the microorganism from the images taken over time;
estimating a microbial growth rate of the microorganism based on data from the digitized area;
and predicting the time required for a number of microorganism to reach a predetermined number that is not acceptable in the food using the estimated microbial growth rate.

5. The method for evaluating shelf life of a food according to claim 4, wherein in digitizing the area of the microorganism is performed by image analysis using a computer.

6. The method for evaluating shelf life of a food according to claim 4 or 5, wherein in estimating the microbial growth rate of the microorganism is performed by calculation using a computer.

7. A system for estimating a microbial growth rate in a food, the system comprising:

an imaging device including a microscope;
a digitization device capable of digitizing an area of a microorganism on a subject from an image of the subject taken by the imaging device over time;
and an estimation device capable of estimating the microbial growth rate of the microorganism from an amount of change in the area over time,
wherein the subject is prepared by placing a hydrophilic membrane filter on a surface of a sample of the food, and inoculating the microorganism on the hydrophilic membrane filter.

8. The system for estimating a microbial growth rate in food according to claim 7, wherein the digitization device is an image analyzer using a computer.

9. The system for estimating a microbial growth rate in food according to claim 7 or 8, wherein the estimation device is a calculation device using a computer.

FIG.1

FIG.2

FIG.3A

$Y_0$

FIG.3B

Yt

FIG.4A

$Y_0P$

FIG.4B

YtP

FIG.5

FIG.6

Number of yeast (cell/g)

$k = 0.4553$

● Actual measurement value

⋯⋯ Predicted value

Actual measurement value is average of three

FIG.7A

$Z_0$

FIG.7B

$Z_t$

FIG.8A

$Z_0P$

FIG.8B

$ZtP$

FIG.9

$$\frac{S_t}{S_0} = e^{0.3174t}$$

FIG.10

Number of yeast (cell/g)

$k = 0.3174$

● Actual measurement value

⋯⋯ Predicted value

Actual measurement value is average of three

FIG.11A

FIG.11B

## INTERNATIONAL SEARCH REPORT

| International application No. |
|---|
| **PCT/JP2023/013171** |

### A. CLASSIFICATION OF SUBJECT MATTER

*C12N 1/16*(2006.01)n; *C12Q 1/06*(2006.01)i; *G01N 33/02*(2006.01)i; *C12M 1/34*(2006.01)i
FI:    C12Q1/06; C12M1/34 D; G01N33/02; C12N1/16

According to International Patent Classification (IPC) or to both national classification and IPC

### B. FIELDS SEARCHED

Minimum documentation searched (classification system followed by classification symbols)

C12N1/16; C12Q1/06; G01N33/02; C12M1/34

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | JP 2018-42502 A (TSUCHIYA KK) 22 March 2018 (2018-03-22) claims, paragraphs [0015]-[0029], [0030]-[0033] (Family: none) | 1-9 |
| A | JP 2004-344087 A (MATSUSHITA ELECTRIC IND CO LTD) 09 December 2004 (2004-12-09) claims, paragraphs [0008], [0033]-[0040], examples (Family: none) | 1-9 |
| A | JP 2010-246442 A (PANASONIC CORP) 04 November 2010 (2010-11-04) claims, paragraphs [0005]-[0008], examples (Family: none) | 1-9 |
| A | JP 2007-195454 A (KAO CORP) 09 August 2007 (2007-08-09) claims, paragraphs [0014]-[0017], examples (Family: none) | 1-9 |

☑ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 May 2023** | **13 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/013171**

### C. DOCUMENTS CONSIDERED TO BE RELEVANT

| Category* | Citation of document, with indication, where appropriate, of the relevant passages | Relevant to claim No. |
|---|---|---|
| A | RODRIGUES, U. M. & KROLL, R. G. Rapid selective enumeration of bacteria in foods using a microcolony epifluorescence microscopy technique. Journal of Applied Bacteriology. 1988, 64(1), pp. 65-78<br>column "abstract" | 1-9 |

Form PCT/ISA/210 (second sheet) (January 2015)

**REFERENCES CITED IN THE DESCRIPTION**

**Patent documents cited in the description**

- JP 2000210099 A

- JP 2007020528 A